# FASCICULE DE BREVET EUROPEEN

(11) **EP 0 392 927 B1**
(45) Date de publication et mention de la délivrance du brevet: **09.08.1995**
(21) Numéro de dépôt: 90401002.2
(22) Date de dépôt: 11.04.1990
(51) Int. Cl.: A61B 17/58, A61F 5/02

(54) **Implant vertébral pour dispositif d'ostéosynthèse**
Wirbelsäulenimplantat für Osteosesyntheseeinrichtung
Vertebral implant for osteosynthesis device

(30) Priorité: 13.04.1989 FR 8904926
(43) Date de publication de la demande: 17.10.1990
(73) Titulaire: SOCIETE DE FABRICATION DE MATERIEL ORTHOPEDIQUE, 62600 Berck/Mer (FR)
(72) Inventeur: Cotrel, Yves Paul Charles, F-75015 Paris (FR)
(74) Mandataire: Martin, Jean-Paul

(56) Documents cités:
- EP-A- 0 227 594
- EP-A- 0 283 373
- FR-A- 2 506 605
- FR-A- 2 545 350

## Description

La présente invention a pour objet un implant vertébral pour dispositif d'ostéosynthèse.

Plus précisément, cet implant est du type comprenant un élément d'ancrage osseux, constitué par une vis ou par une lame recourbée formant crochet, un corps présentant un canal adapté pour être traversé par une tige, et une première vis de solidarisation de l'implant avec la tige, cette vis ayant son axe perpendiculaire à la tige contre laquelle elle est serrée, soit à travers le corps, soit à travers un bloqueur pouvant coulisser sur la tige et venir se loger dans le corps ; l'implant comprend au moins une seconde vis de blocage de la tige traversant le corps latéralement à la première vis et engagée dans un trou taraudé (EP-A-0283373).

Il est connu, dans des dispositifs d'ostéosynthèse rachidienne, de mettre en oeuvre des tiges métalliques à aspérités de surface qui permettent la fixation de crochets, comme décrit par le brevet français 83 07 450 (2 545 350) ou de vis comme décrit par le brevet français 87 03 485. Cette fixation peut être effectuée dans toutes les positions de niveau, dans tous les sens et dans toutes les directions, au moyen d'une ou deux vis de blocage vissées à travers le corps de l'implant sur la tige. Le corps des crochets et des vis est, soit fermé autour de son canal de passage de la tige, soit à ouverture postérieure, soit à ouverture latérale débouchant dans le canal.

Les ouvertures permettent l'insertion directe de la tige dans le canal du corps de l'implant. Dans le cas d'un implant à ouverture postérieure, la fixation tige-implant est obtenue au moyen d'une pièce intermédiaire dite bloqueur, formée d'un anneau dont une moitié est conique, et l'autre moitié cylindrique, surmonté d'un corps carré taraudé pour le passage d'une vis de blocage et la fixation en rotation (brevet précité 83 07 450).

La tige munie de son bloqueur est insérée dans le canal de l'implant qui vient s'empaler sur le bloqueur, assurant après vissage de ce dernier une fixation implant-tige. Pour éviter tout déplacement de l'implant sur son bloqueur du côté de l'extrémité conique de celui-ci, une pièce complémentaire dite verrou de sûreté, est nécessaire. Elle est constituée d'un anneau à ouverture latérale de passage de la tige, munie de deux bras enserrant partiellement le corps de l'implant et vissée directement sur la tige.

Ce dispositif antérieur est constitué essentiellement de trois pièces (crochet, verrou et bloqueur) relativement encombrantes. Il en résulte une certaine difficulté de montage, en particulier à la jonction des régions lombaires et sacrées où un faible espace reste disponible en raison des conditions anatomiques.

De plus dans ce dispositif antérieur, un risque de glissement éventuel du bloqueur par rapport au crochet de l'implant ne peut être totalement écarté, bien que ce soit là précisément la fonction du verrou de sûreté.

L'invention a donc pour but de réaliser un implant dans lequel ces inconvénients sont éliminés, et qui assure une fixation de la tige dans le corps de l'implant plus solide que celle obtenue dans le dispositif du brevet français 83 07 450.

Suivant l'invention, l'implant vertébral est caractérisé par les moyens mentionnés à la partie caractérisante de la revendication 1.

Selon un mode de réalisation possible, deux trous taraudés ayant leurs axes inclinés sur l'axe de la première vis sont ménagés dans le corps de chaque côté de la première vis, pour recevoir des vis de blocage correspondantes.

Si le corps est du type fermé, l'implant ne comporte pas de bloqueur, et les deux ou trois vis précitées traversent des trous taraudés ménagés dans le corps. Si le corps est du type à ouverture postérieure ou latérale, débouchant dans le canal, l'implant est équipé d'un bloqueur qui peut venir se loger à l'intérieur du corps et est pourvu de la première vis centrale, de part et d'autre de laquelle sont placées les deux vis latérales obliques, qui sont vissées directement sur la tige à travers des trous percés dans les branches du corps et dans le bloqueur.

Avantageusement, la tige peut être fixée dans l'implant par trois vis, à savoir les deux vis latérales obliques et la vis du bloqueur.

Cet implant ne comporte donc plus de verrou, contrairement au dispositif du brevet antérieur précité, tout en garantissant une fixation sur la tige dans tous les sens et avec une plus grande solidité que précédemment. L'encombrement de l'implant ainsi réalisé est donc notablement réduit, ce qui facilite sa mise en place par le chirurgien, en particulier dans les régions lombaires et sacrées.

D'autres particularités et avantages de l'invention apparaîtront au cours de la description qui va suivre, faite en référence aux dessins annexés qui en illustrent plusieurs formes de réalisation à titre d'exemples non-limitatifs.

La figure 1 est une vue en élévation d'une première forme de réalisation de l'implant vertébral selon l'invention, monté sur la tige correspondante.

La figure 2 est une vue en élévation selon la flèche K de la Fig.1.

La figure 3 est une vue de dessus de l'implant des Fig.1 et 2.

La figure 4 est une vue en élévation d'une seconde forme de réalisation de l'implant selon l'invention.

La figure 5 est une vue en élévation dans la direction de la flèche L de la Fig.4, et montre également, en traits mixtes, une variante de réalisation possible de cet implant.

La figure 6 est une vue de dessus de l'implant des Fig.4 et 5.

La figure 7 est une vue en élévation partielle analogue à la Fig.1 d'une troisième forme de réalisation de l'implant selon l'invention.

La figure 8 est une vue en élévation d'une quatrième forme de réalisation de l'implant selon l'invention, dans lequel l'élément d'ancrage osseux est un crochet.

La figure 9 est une vue en élévation latérale de l'implant de la Fig.8.

Les figures 10 et 11 sont des vues analogues aux Fig.8 et 9 d'une variante d'implant à crochet.

La figure 12 est une vue en élévation éclatée d'une cinquième forme de réalisation de l'implant selon l'invention, dans la direction axiale de la flèche L de la Fig.13.

La figure 13 est une vue en élévation de l'implant de la Fig.12 dans une direction perpendiculaire au plan de cette Fig.12.

La figure 14 est une vue de dessus en plan du bloqueur de l'implant des Fig.12 et 13.

La figure 15 est une vue en élévation d'un bloqueur selon une sixième forme de réalisation de l'implant dans la direction de la flèche M de la Fig.16.

La Figure 16 est une vue en élévation du bloqueur de la Fig.15 dans un plan perpendiculaire à celui de cette Fig.15.

La figure 17 est une vue de dessus en plan du bloqueur des Fig.15 et 16.

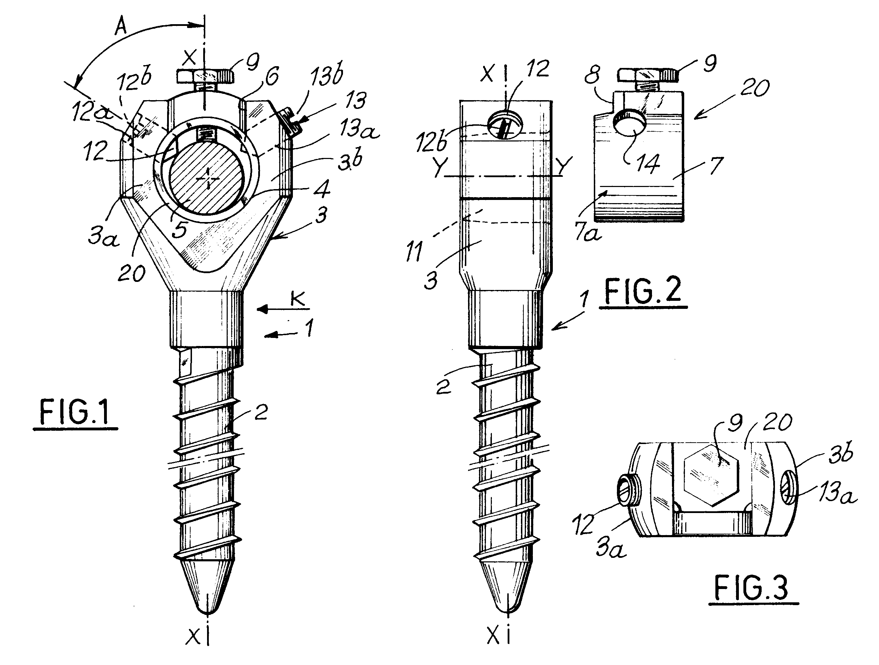

L'implant vertébral représenté aux Fig.1 à 3 est destiné à un dispositif d'ostéosynthèse pour la chirurgie du rachis.

Cet implant 1 comprend un élément d'ancrage osseux 2, ici constitué par une vis de dimensions et de filetage appropriés. L'implant 1 comprend également un corps 3 monopièce avec la vis 2 et qui présente un canal central 4 adapté pour être traversé par une tige 5 dont la surface peut être lisse ou à aspérités, par exemple recouverte de pointes de diamant. Le corps 3 est du type à ouverture postérieure 6, coaxiale à l'axe XX de l'implant 1, et délimitée par deux branches 3a et 3b dont les surfaces intérieures forment des rives pour le canal 4.

L'implant 1 est muni d'un bloqueur annulaire 20 constitué d'une bague cylindrique 7, d'une extension radiale 8 faisant saillie de la bague 7, et d'une vis 9 pouvant être vissée dans un trou taraudé de l'extension 8 pour venir s'appliquer sur la surface de la tige 5. Le diamètre intérieur de la bague 7 est légèrement supérieur au diamètre de la tige 5 afin que le bloqueur 20 puisse coulisser sur celle-ci, et ce bloqueur est dimensionné pour pouvoir venir s'engager dans le canal 4 avec son extension 8 placée entre les extrémités des branches 3a, 3b dans l'ouverture postérieure 6. La bague 7 est pourvue d'une partie avant tronconique 7a adaptée pour venir s'emboîter en butée contre une partie tronconique complémentaire 11 du canal 4.

L'implant 1 est équipé de moyens de blocage sur la tige 5 complémentaires de la première vis centrale 9. Ces moyens sont constitués, dans l'exemple décrit, par une seconde et une troisième vis 12, 13 traversant les branches respectives 3a, 3b du corps 3 latéralement à la première vis 9 et de chaque côté de celle-ci, par rapport à l'axe de laquelle elles sont inclinées d'un angle A approprié. Les vis 12, 13 sont vissées dans des trous taraudés correspondants 12a, 13a d'inclinaison A sur l'axe XX de l'implant 1 et de la vis 9. Les deux vis de blocage 12 et 13 sont situées sensiblement dans un même plan perpendiculaire à l'axe YY du canal 4 et de la tige 5, c'est-à-dire le plan de la Fig.1.

En variante, l'implant 1 pourrait n'être équipé que d'une vis 12 ou 13 associée à un unique trou taraudé, ou encore seule l'une des vis 12 et 13 peut être mise en place dans l'implant 1, dans certains cas de mise en place difficile dans des régions du rachis où l'espace est restreint. Afin de permettre leur vissage, des empreintes en creux 12b, 13b profilées de manière appropriée pour recevoir un outil de vissage, sont formées dans les têtes des vis 12 et 13.

La mise en place de l'implant 1 qui vient d'être décrit sur sa tige 5 s'effectue de manière très simple : le bloqueur 20 étant préalablement monté librement coulissant sur la tige 5, celle-ci est introduite dans le canal 4 par l'ouverture postérieure 6. Puis on fait coulisser le bloqueur 20 jusqu'à ce qu'il pénètre entre les branches 3a et 3b et que sa partie conique 7a vienne en butée contre la partie complémentaire 11. On visse alors successivement les vis 9 et 12, 13, ces deux dernières traversant des trous respectifs tels que 14, symétriques par rapport à l'axe XX.

L'encombrement de cet implant dans la direction de la tige 5 est fortement diminué par rapport à celui de l'implant décrit au brevet français 83 07 450, en particulier grâce à la suppression du verrou. Par ailleurs, il convient de noter que les parois ou branches 3a, 3b du corps 3 sont épaissies par rapport à celles du corps du crochet décrit au brevet français précité, afin d'accroître leur résistance mécanique. Elles sont également arrondies afin de diminuer ou supprimer des angles vifs susceptibles de blesser les tissus du patient.

La réduction d'encombrement de l'implant 1 est particulièrement avantageuse dans les cas où son volume peut rendre l'instrumentation difficile ou impossible, pour des raisons anatomiques, telles qu'au niveau du sacrum. Enfin, si l'on utilise des vis 12, 13 sans tête, présentant des empreintes 12b, 13b par exemple hexagonales du type M3HC (brevet français 87 16 209) il est possible de retirer le matériel implanté par dévissage des deux vis 12 et 13 ainsi que de la vis centrale 9.

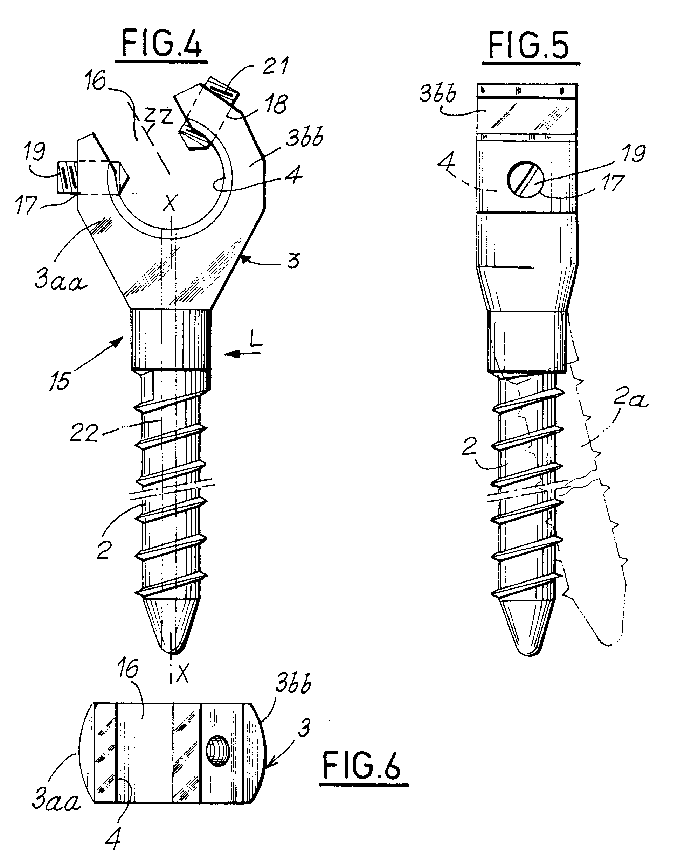

L'implant 15 représenté aux Fig.4 à 6 diffère de l'implant 1 par le fait que l'ouverture postérieure 6 est ici remplacée par une ouverture latérale 16 dont l'axe ZZ est incliné sur l'axe XX de l'implant 15 d'un angle approprié, par exemple 25 ou 30°. (Ces valeurs n'étant naturellement données qu'à titre indicatif non limitatif et pouvant très largement varier). L'ouverture 16 et le canal 4 sont donc ici délimités par des branches latérales 3aa et 3bb de longueurs inégales, la branche 3aa étant plus courte que la branche 3bb. De ce fait les deux trous taraudés 17, 18 ménagés dans les branches 3aa et 3bb et leurs vis correspondantes 19, 21, inclinées sur l'axe ZZ et symétriques par rapport à celui-ci, présentent des inclinaisons différentes sur l'axe XX. Toutefois, la mise en oeuvre de l'implant 15, qui comprend un bloqueur non représenté et similaire au bloqueur 6, se fait de la même façon que celle de l'implant 1.

Comme dans la réalisation précédente, l'implant 15 peut ne comporter qu'un seul trou taraudé 17 ou 18, ou en comporter deux, mais n'être mis en oeuvre qu'avec une seule des vis 19 et 21.

Selon une variante d'exécution possible, le fond du canal 4 peut être relié à l'extrémité de la vis 2 par un conduit axial 22 de guidage d'implantation de la vis et du corps, sur une tige non représentée, enfilée dans ce conduit 22.

Selon une autre variante possible (Fig.5), la vis 2 est remplacée par une vis 2a inclinée d'un angle approprié sur l'axe XX et formant donc avec le corps 3 un angle obtus. L'implant ainsi réalisé est adapté à une utilisation dans le sacrum pour une fixation oblique.

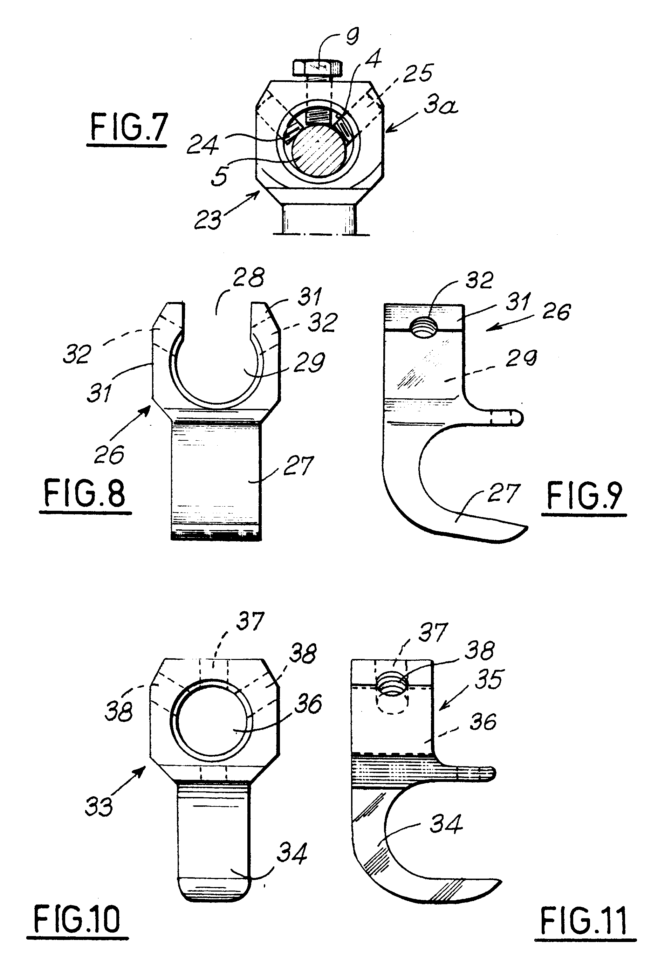

La troisième forme de réalisation de l'implant 23 représentée à la Fig.7, comprend un corps fermé 3a, démuni par conséquent de bloqueur. La vis centrale 9 est introduite ici à travers le corps 3a en position centrale comme dans les réalisations précédentes, et vissée sur la tige 5, sur laquelle viennent également s'appliquer deux vis obliques 24, 25 symétriques par rapport à la vis 9 et qui traversent des trous taraudés correspondants du corps 3a.

Tous ces modes de réalisation de l'implant selon l'invention ont en commun le gain d'encombrement et une plus grande facilité de montage, ainsi qu'une meilleure solidité de fixation que les implants connus, décrits dans les brevets précités.

L'implant 26 des Fig. 8 et 9 est un crochet formé d'une lame recourbée 27 et d'un corps à ouverture postérieure 28 et canal 29 délimités par deux branches 31 percées de deux trous taraudés obliques 32 de réception de vis non représentées (ni le bloqueur, similaire au bloqueur 20).

L'implant des Fig. 10 et 11 est également un crochet 33 à lame 34 mais son corps 35 est fermé. Il est percé d'un canal 36 et de trois trous taraudés 37, 38 de passage des vis.

Dans une autre variante, les parties tronconiques 7a et 11 peuvent être remplacées par toutes parties mâle et femelle appropriées.

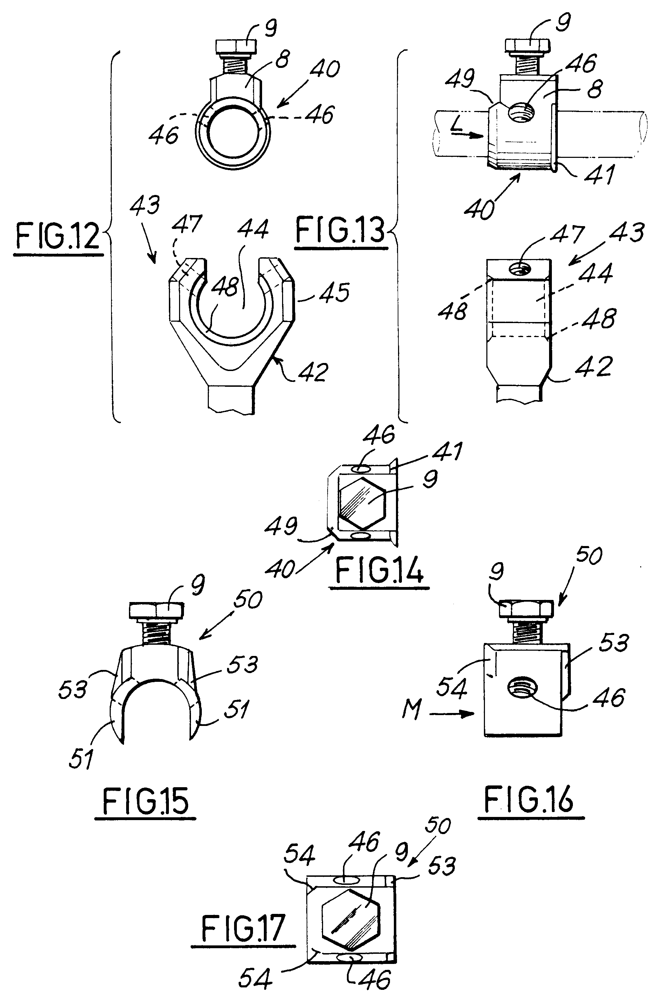

Dans la forme de réalisation des Fig.12 à 14, le bloqueur 40 est annulaire et muni à l'une de ses extrémités d'une butée 41 saillant radialement. Cette butée 41 peut ainsi se présenter sous la forme d'une collerette adaptée pour venir en appui sur le corps 42 de l'implant 43 et plus précisément sur l'une des extrémités de son canal 44, le corps 42 étant du type ouvert avec son canal 44 délimité par deux branches latérales 45. La butée 41 a pour fonction, en venant en appui contre le bord d'entrée du canal 44, de positionner correctement le bloqueur 40 dans le corps 42, afin que les trous taraudés 46 de passage des vis latérales dans le bloqueur 40 viennent en correspondance précise avec les trous 47 du corps 42.

Des chanfreins 48 peuvent avantageusement être ménagés sur les bords opposés des ouvertures du canal 44, pour faciliter l'introduction du bloqueur 40 dans le corps 42. Dans ce cas l'extrémité du bloqueur 40 opposée à son extrémité portant la butée 41 présente un chanfrein complémentaire 49 tandis que la butée 41 est profilée en un biseau ou chanfrein conique pour pouvoir s'appliquer sur l'un des chanfreins 48 d'entrée du canal 44.

Si aucun chanfrein 48 n'est ménagé sur le corps 42, la butée 41 peut être par exemple circulaire, ou à section carrée.

Le canal 44 étant cylindrique sur toute sa longueur et élargi à chacune de ses extrémités d'un chanfrein 48, il devient possible d'introduire le bloqueur 40 dans le corps 42 par l'une ou l'autre des ouvertures du canal 44.

Le bloqueur 50 représenté aux Fig.15 à 17 est réalisé sous la forme d'un cavalier à deux pattes 51 qui délimitent entre elles un canal 52 ouvert radialement dans la direction opposée à la vis centrale 9. Les pattes latérales 51 et le profil semicirculaire du canal 52 sont adaptés pour coiffer la tige (non représentée) en l'enserrant entre les pattes 51. Le bloqueur 50 est en outre muni d'une butée saillant radialement, se présentant dans cet exemple sous la forme de deux excroissances latérales 53 adaptées pour venir en appui sur le corps de l'implant, afin de positionner de manière adéquate par rapport à ce dernier le bloqueur 50. On remarque que les excroissances 53 sont réalisées de manière que leurs surfaces viennent tangenter les extrémités des pattes 51. Par ailleurs, un biseau 54 est ménagé sur chaque côté de l'extrémité du bloqueur 50 opposée à son extrémité portant les excroissances de butée 53. Les parties biseautées 54 facilitent l'introduction du bloqueur 50 dans le corps de l'implant.

Grâce à la conformation du bloqueur 50 en cavalier avec un canal 52 ouvert radialement, il devient possible de mettre en place ce bloqueur sur la tige correspondante après introduction de celle-ci dans le corps de l'implant, en la coiffant par ce bloqueur 50. En effet, lorsque le bloqueur est constitué d'un cylindre fermé, il est évidemment nécessaire de l'enfiler sur la tige préalablement à l'introduction de cette dernière dans le corps de l'implant.

## Revendications

1. Implant vertébral (1, 15, 23) pour dispositif d'ostéosynthèse, comprenant un élément d'ancrage osseux (2), un corps (3, 3a) présentant un canal (4) adapté pour être traversé par une tige (5) et une première vis (9) de solidarisation de l'implant avec la tige, cette vis (9) ayant son axe perpendiculaire à la tige (5) contre laquelle elle est serrée à travers le corps (3, 3a) ou à travers un bloqueur (20) pouvant coulisser sur la tige et venir se loger dans le corps, au moins une seconde vis (12, 13) de blocage de la tige (5), traversant le corps (3) latéralement à la première vis (9) et engagée dans un trou taraudé (12a, 13a) caractérisé en ce que ladite seconde vis est inclinée sur l'axe (XX) de ladite première vis, ces deux vis (9, 12 ou 13) étant situées sensiblement dans un plan perpendiculaire à l'axe (YY) du canal (4) et de la tige (5).

2. Implant selon la revendication 1, caractérisé en ce que deux trous taraudés (12a, 13a) ayant leurs axes inclinés sur l'axe (XX) de la première vis (9) sont ménagés dans le corps (3) de chaque côté de ladite première vis (9) pour recevoir des vis de blocage (12, 13; 24, 25) correspondantes.

3. Implant selon l'une des revendications 1 et 2, dans lequel le corps (3) présente une ouverture postérieure (6) ou latérale (16) débouchant dans le canal (4) et délimitée par deux branches (3a, 3b; 3aa, 3bb) formant des rives du canal, dans lesquelles est ménagé au moins un trou taraudé (12a, 13a; 17, 18) de réception d'une vis latérale (12, 13; 19, 21), caractérisé en ce qu'il comprend un bloqueur annulaire (20) pouvant coulisser sur la tige (5) et être introduit par ladite ouverture postérieure (6) ou latérale (16) dans le canal (4), la première vis centrale (9) traversant le bloqueur (20) et la ou les vis latérales traversant lesdites branches ainsi que le bloqueur.

4. Implant selon la revendication 3, caractérisé en ce que le bloqueur (20) est pourvu d'une partie mâle (7a) adaptée pour venir s'emboîter en butée contre une partie femelle complémentaire (11) du canal (4) du corps (3), ainsi que d'une extension radiale (8) pouvant être introduite dans l'ouverture (6 ou 16) du corps (3).

5. Implant selon l'une des revendications 1 à 4, dans lequel l'élément d'ancrage est une vis (2) perpendiculaire à l'axe (YY) du canal (4), caractérisé en ce que le fond du canal (4) est relié à l'extrémité de la vis (2) par un conduit axial (22) de guidage d'implantation, le corps (3) présentant alors une ouverture postérieure (6) ou latérale (16) délimitée par deux branches (3a, 3b; 3aa, 3bb) dans l'une au moins desquelles est agencée un trou taraudé incliné (17 ou 18) de réception d'une vis de blocage (19 ou 21).

6. Implant selon l'une des revendications 1 à 5, caractérisé en ce que l'élément d'ancrage est une vis (2a) formant un angle obtus avec le corps (3).

7. Implant selon l'une des revendications 1 à 4, caractérisé en ce que l'élément d'ancrage est une lame recourbée (27, 34).

8. Implant selon l'une des revendications 1 à 3, caractérisé en ce que le bloqueur (40) est annulaire et muni d'un butée (41) saillant radialement et adaptée pour venir en appui sur le corps de l'implant afin de positionner de manière adéquate le bloqueur par rapport au corps, cette butée étant par exemple une collerette.

9. Implant selon la revendication 8, caractérisé en ce que des chanfreins (48) sont ménagés sur les bords des ouvertures du canal (44) du corps (42) pour faciliter l'introduction du bloqueur (40) dans le corps, et l'extrémité (49) du bloqueur opposée à sa butée (41) est chanfreinée de manière complémentaire tandis que ladite butée (41) est profilée pour s'appliquer sur l'un desdits chanfreins (48).

10. Implant selon l'une des revendications 1 et 2, caractérisé en ce qu'il comporte un bloqueur réalisé sous la forme d'un cavalier (50) à deux pattes (51), adapté pour coiffer la tige en l'enserrant entre les pattes, lesquelles délimitent entre elles un canal (52) ouvert radialement, de telle sorte que le cavalier (50) peut être mis en place radialement sur la tige après positionnement de cette dernière dans le corps.

11. Implant selon la revendication 10, caractérisé en ce que le bloqueur (50) est pourvu d'une butée (53) saillant radialement et adaptée pour venir en appui sur le corps afin de positionner de manière adéquate le bloqueur par rapport au corps.

## Claims

1. Vertebral implant (1, 15, 23) for an osteosynthesis device, comprising a bone anchoring element (2), a body (3, 3a) with a channel (4) suitable for having a rod (5) pass through it and a first screw (9) for fixing the implant to the rod, the axis of this screw (9) being perpendicular to the rod (5) against which it is tightened through the body (3, 3a) or through a locking device (20) able to slide on the rod and be housed in the body, at least one second screw (12, 13) for locking the rod (5), passing through the body (3) laterally to the first screw (9) and engaged in a threaded hole (12a, 13a), characterised in that the said second screw is inclined to the axis (XX) of the said first screw, these two screws (9, 12 or 13) being situated substantially in a plane perpendicular to the axis (YY) of the channel (4) and rod (5).

2. Implant according to Claim 1, characterised in that two threaded holes (12a, 13a) having their axes inclined to the axis (XX) of the first screw (9) are formed in the body (3) on each side of the said first screw (9) in order to receive corresponding locking screws (12, 13; 24, 25).

3. Implant according to one of Claims 1 and 2, in which the body (3) has a rear (6) or lateral (16) opening, opening out into the channel (4) and defined by two arms (3a, 3b; 3aa, 3bb) forming edges of the channel, in which at least one threaded hole (12a, 13a; 17, 18) for receiving a lateral screw (12, 13; 19, 21) is formed, characterised in that it comprises an annular locking device (20) able to slide on the rod (5) and be inserted through the said rear (6) or lateral (16) opening into the channel (4), the first central screw (9) passing through the locking device (20) and the lateral screw or screws passing through the said arms and the locking device.

4. Implant according to Claim 3, characterised in that the locking device (20) is provided with a male part (7a) suitable for fitting in abutment against a complementary female part (11) of the channel (4) in the body (3), and a radial extension (8) able to be inserted into the opening (6 or 16) in the body (3).

5. Implant according to one of Claims 1 to 4, in which the anchoring element is a screw (2) perpendicular to the axis (YY) of the channel (4), characterised in that the bottom of the channel (4) is connected to the end of the screw (2) by an axial pipe (22) for guiding the fitting, the body (3) then having a rear (6) or lateral (16) opening defined by two arms (3a, 3b; 3aa, 3bb) in at least one of which is arranged an inclined threaded hole (17 or 18) for receiving a locking screw (19 or 21).

6. Implant according to one of Claims 1 to 5, characterised in that the anchoring element is a screw (2a) forming an obtuse angle with the body (3).

7. Implant according to one of Claims 1 to 4, characterised in that the anchoring element is a curved blade (27, 34).

8. Implant according to one of Claims 1 to 3, characterised in that the locking device (40) is annular and provided with a stop (41) projecting radially and suitable for coming to bear on the body of the implant so as to position the locking device suitably in relation to the body, this stop being, for example, a collar.

9. Implant according to Claim 8, characterised in that bevels (48) are formed on the edges of the openings of the channel (44) in the body (42) in order to facilitate the insertion of the locking device (40) into the body, and the end (49) of the locking device opposite its stop (41) is bevelled in a complementary manner, while the said stop (41) is profiled so as to be applied to one of the said bevels (48).

10. Implant according to one of Claims 1 and 2, characterised in that it has a locking device produced in the form of a bracket (50) with two lugs (51), suitable for fitting on the rod by gripping it between the lugs, which define between them a radially open channel (52), such that the bracket (50) can be positioned radially on the rod after the latter has been positioned in the body.

11. Implant according to Claim 10, characterised in that the locking device (50) is provided with a stop (53) projecting radially and suitable for coming to bear on the body so as to position the locking device suitably in relation to the body.

## Patentansprüche

1. Wirbelsäulenimplantat (1, 15, 23) für eine Osteosynthese-Einrichtung, mit einem Knochenverankerungselement (2), mit einem Grundkörper (3, 3a), der einen Kanal (4) ausbildet, welcher dazu ausgestaltet ist, um von einer Stange (5) und von einer ersten Schraube (9) durchquert zu werden, um das Implantat an der Stange zu befestigen, wobei die Schraube (9) mit ihrer Achse senkrecht zur Stange (5) verläuft, gegen welche sie festgeschraubt ist, indem sie sich durch den Grundkörper (3, 3a) oder durch ein Klemmelement (20) erstreckt, welches auf der Stange verschiebbar ist und sich in den Grundkörper einschieben läßt, und mit mindestens einer zweiten Befestigungsschraube (12, 13) für die Stange (5), die sich benachbart zur ersten Schraube (9) durch den Grundkörper (3) erstreckt und in eine Gewindebohrung (12a, 13a) eingreift, **dadurch gekennzeichnet**, daß die zweite Schraube bezüglich der Achse (XX) der ersten Schraube geneigt ist und daß die beiden Schrauben (9, 12 oder 13) genau in einer Ebene angeordnet sind, die senkrecht zur Achse (YY) des Kanals (4) und der Stange (5) verläuft.

2. Implantat nach Anspruch 1, **dadurch gekennzeichnet**, daß zwei Gewindebohrungen (12a, 13a), deren Achsen bezüglich der Achse (XX) der ersten Schraube (9) geneigt sind, im Grundkörper (3) an jeder Seite der ersten Schraube (9) ausgebildet sind, um die zugehörigen Befestigungsschrauben (12, 13; 24, 25) aufzunehmen.

3. Implantat nach einem der Ansprüche 1 und 2, wobei im Grundkörper (3) eine hintere (6) oder seitliche (16) Öffnung vorgesehen ist, welche in den Kanal (4) einmündet und welche durch zwei die Randbereiche des Kanals bildende Abschnitte (3a, 3b; 3aa, 3bb) begrenzt ist, in denen mindestens eine Gewindebohrung (12a, 13a; 17, 18) zur Aufnahme einer seitlichen Schraube (12, 13; 19, 21) ausgebildet ist, **dadurch gekennzeichnet**, daß ein ringförmiges Klemmelement (20) vorgesehen ist, das auf der Stange (5) verschiebbar ist und sich über die hintere (6) oder seitliche (16) Öffnung in den Kanal (4) einschieben läßt, wobei sich die erste mittlere Schraube (9) durch das Klemmelement (20) erstreckt und wobei sich die seitliche Schraube bzw. Schrauben sowohl durch die Abschnitte als auch durch das Klemmelement erstreckt bzw. erstrecken.

4. Implantat nach Anspruch 3, **dadurch gekennzeichnet**, daß das Klemmelement (20) mit einem vorstehenden Bereich (7a), der dazu ausgestaltet ist, um passend mit einem entsprechend vertieften Bereich (11) im Kanal (4) des Grundkörpers (3) in Anlage zu kommen, und mit einem sich radial erstreckenden Vorsprung (8) versehen ist, der in die Öffnung (6 oder 16) des Grundkörpers (3) eingeschoben werden kann.

5. Implantat nach einem der Ansprüche 1 bis 4, wobei das Verankerungselement eine senkrecht zur Achse (YY) des Kanals (4) verlaufende Schraube (2) ist, **dadurch gekennzeichnet**, daß sich am Boden des Kanals (4) am Ende der Schraube (2) eine axiale Röhre (22) anschließt, um die Implantation zu führen, und daß im Grundkörper (3) eine hintere (6) oder seitliche (16) Öffnung vorgesehen ist, die durch zwei Abschnitte (3a, 3b; 3aa, 3bb) begrenzt ist, von denen mindestens einer mit einer geneigten Gewindebohrung (17 oder 18) versehen ist, um eine Befestigungsschraube (19 oder 21) aufzunehmen.

6. Implantat nach einem der Ansprüche 1 bis 5, **dadurch gekennzeichnet**, daß das Verankerungselement eine Schraube (2a) ist, die mit dem Grundkörper (3) einen stumpfen Winkel bildet.

7. Implantat nach einem der Ansprüche 1 bis 4, **dadurch gekennzeichnet**, daß das Verankerungselement ein gekrümmter Arm (27, 34) ist.

8. Implantat nach einem der Ansprüche 1 bis 3, **dadurch gekennzeichnet**, daß das Klemmelement (40) ringförmig ist, einen sich radial erstreckenden Anschlag (41) aufweist und dazu ausgestaltet ist, um am Grundkörper des Implantats in Anlage zu kommen, um das Klemmelement bezüglich des Grundkörpers genau ausrichten zu können, wobei der Anschlag beispielsweise ein Kragenansatz ist.

9. Implantat nach Anspruch 8, **dadurch gekennzeichnet**, daß an den Rändern der Öffnungen des Kanals (44) des Grundkörpers (42) Abschrägungen (48) ausgebildet sind, um das Einschieben des Klemmelementes (40) in den Grundkörper zu erleichtern, und daß das dem Anschlag (41) gegenüberliegende Ende (49) des Klemmelementes auf entsprechende Weise abgeschrägt ist, während der Anschlag (41) dazu ausgestaltet ist, um an einer der Abschrägungen (48) anzuliegen.

10. Implantat nach einem der Ansprüche 1 und 2, **dadurch gekennzeichnet**, daß ein Klemmelement vorgesehen ist, welches durch zwei unterhalb der Gestalt eines Schiebers (50) ausgeformte Klauen (51) gebildet ist, die dazu ausgestaltet sind, um die Stange zu überdecken, indem diese zwischen den Klauen eingespannt ist, welche einen zwischen ihnen liegenden Kanal (52) begrenzen, der in radialer Richtung derart geöffnet ist, daß der Schieber (50) in radialer Richtung auf die Stange aufgesetzt werden kann, nachdem diese letztere in den Grundkörper eingesetzt ist.

11. Implantat nach Anspruch 10, **dadurch gekennzeichnet**, daß das Klemmelement (50) einen in radialer Richtung vorstehenden Anschlag (53) aufweist und dazu ausgestaltet ist, um am Grundkörper in Anlage zu kommen, um das Klemmelement bezüglich des Grundkörpers genau positionieren zu können.
